# EUROPEAN PATENT APPLICATION

(11) **EP 4 524 188 A1**
(43) Date of publication of application: **19.03.2025**
(21) Application number: 23936789.9
(22) Date of filing: 23.11.2023
(51) Int. Cl.: C08J 11/10, C08G 64/30, C07C 37/055, C07C 37/84, C07C 39/16

(54) **MONOMER COMPOSITION FOR SYNTHESIZING RECYCLED PLASTIC, METHOD FOR PREPARING SAME, AND RECYCLED PLASTIC AND MOLDED ARTICLE USING SAME**

(30) Priority: 28.07.2023 KR 20230098842
(71) Applicant: LG CHEM, LTD., Yeongdeungpo-gu Seoul 07336 (KR)
(72) Inventor: KIM, Jeongnam, Daejeon 34122 (KR); KIM, Minju, Daejeon 34122 (KR); LEE, Jeongbin, Daejeon 34122 (KR); HWANG, Jihwan, Daejeon 34122 (KR); HONG, Mooho, Daejeon 34122 (KR); PARK, Eunju, Daejeon 34122 (KR)
(74) Representative: Goddar, Heinz J.
(86) International application number: PCT/KR2023/018954
(87) International publication number: WO 2025/028723

(57) **Abstract**

The present invention relates to a monomer composition for synthesizing recycled plastic, a preparation method thereof, and recycled plastic, molded product using the same. The monomer composition for synthesizing recycled plastic comprises an aromatic diol compound, wherein a ratio of aromatic diol compound derivative impurities according to Equation 1 is 0.3% or less, wherein the melting point is 156.4°C or more, and wherein the monomer composition is recovered from a polycarbonate-based resin, a preparation method thereof, and a recycled plastic and a molded product using the same.

## Description

### [TECHNICAL FIELD]

### CROSS-REFERENCE TO RELATED APPLICATION(S)

This application claims the benefit of Korean Patent Application No. 10-2023-0098842 filed on July 28, 2023 with the Korean Intellectual Property Office, the disclosure of which is incorporated herein by reference in its entity.

The present invention relates to a monomer composition for synthesizing recycled plastic that can realize high purity and excellent color quality even though it is recovered through recycling by chemical decomposition of a polycarbonate-based resin, and achieve improvement in efficiency of the recovering process, a preparation method thereof, and a recycled plastic and molded product using the same.

### [BACKGROUND]

Polycarbonate is a thermoplastic polymer and is a plastic having excellent characteristics such as excellent transparency, superior ductility, and relatively low production costs.

Although polycarbonate is widely used for various purposes, environmental and health concerns during waste treatment have been continuously raised.

Currently, a physical recycling method is carried out, but in this case, a problem accompanying the deterioration of the quality occurs, and thus, research on the chemical recycling of polycarbonate is underway.

Chemical decomposition of polycarbonate refers to obtaining an aromatic diol compound as a monomer (e.g., bisphenol A (BPA)) through decomposition of polycarbonate, and then utilizing it again in polymerization to obtain a high-purity polycarbonate.

Meanwhile, in order to purify aromatic diol compounds obtained through decomposition of polycarbonate and ensure high purity, conventionally, the process of redissolving and recrystallizing them in an organic solvent such as toluene has been advanced, but there are limitations that require management because the kind of solvents increases and they are toxic substances.

### [DETAILED DESCRIPTION OF THE INVENTION]

### [Technical Problem]

It is an object of the present invention to provide a monomer composition for synthesizing recycled plastic that can realize high purity and excellent color quality even though it is recovered through recycling by chemical decomposition of a polycarbonate-based resin, and achieve improvement in efficiency of the recovering process.

It is another object of the present invention to provide a method for preparing the monomer composition for synthesizing recycled plastic, and a recycled plastic and a molded product using the monomer composition for synthesizing recycled plastic.

### [Technical Solution]

In order to achieve the above object, provided herein is a monomer composition for synthesizing recycled plastic, comprising an aromatic diol compound, wherein a ratio of aromatic diol compound derivative impurities according to the following Equation 1 is 0.3% or less, wherein the melting point is 156.4°C or more, and wherein the monomer composition is recovered from a polycarbonate-based resin. Ratio of aromatic diol compound derivative impurities (%) = (Peak area of aromatic diol compound derivative measured by a liquid chromatography / Total peak area measured by a liquid chromatography) X 100.

Also provided herein is a method for preparing a monomer composition for synthesizing recycled plastic, the method comprising the steps of: subjecting a polycarbonate-based resin to a depolymerization reaction; separating a carbonate-based compound from the depolymerization reaction product; dissolving the depolymerization reaction product from which the carbonate-based compound has been separated in a solvent containing the separated carbonate-based compound; and recrystallizing the aromatic diol compound from the dissolved solution.

Further provided herein is a method for preparing a monomer composition for synthesizing recycled plastic, the method comprising the steps of: subjecting a polycarbonate-based resin to a depolymerization reaction; dissolving the depolymerization reaction product in a solvent containing the carbonate-based compound; and recrystallizing the aromatic diol compound from the dissolved solution.

Further provided herein is a recycled plastic comprising a reaction product of the monomer composition for synthesizing recycled plastic and a comonomer.

Further provided herein is a molded product comprising the recycled plastic.

Below, a monomer composition for synthesizing recycled plastic, a preparation method thereof, and a recycled plastic, and molded product using the same according to specific embodiments of the present invention will be described in more detail.

Unless explicitly stated herein, the technical terms used herein are for the purpose of describing specific embodiments only and is not intended to limit the scope of the invention.

The singular forms "a," "an" and "the" used herein are intended to include plural forms, unless the context clearly indicates otherwise.

The 'pH' as used herein means a hydrogen ion concentration (pH), which is a numerical value indicating the acidity and alkalinity of a material. The pH can be determined from a value expressed by taking the reciprocal of the logarithmic dissociation concentration of hydrogen ions, and is used as a measure of the strength of acids and bases of a material.

As used herein, a derivative compound means a compound in which when a certain organic compound is used as a matrix compound, it undergoes changes such as introduction of functional groups, oxidation, reduction, and atom substitution within the limits that do not significantly change the structure and properties of the matrix compound.

As used herein, the term "substituted or unsubstituted" means being unsubstituted or substituted with one or more substituent groups selected from the group consisting of deuterium; a halogen group; a cyano group; a nitro group; a hydroxy group; a carbonyl group; an ester group; an imide group; an amino group; a primary amino group; a carboxy group; a sulfonic acid group; a sulfonamide group; a phosphine oxide group; an alkoxy group; an aryloxy group; an alkylthioxy group; an arylthioxy group; an alkylsulfoxy group; an arylsulfoxy group; a silyl group; a boron group; an alkyl group; a cycloalkyl group; an alkenyl group; an aryl group; an aralkyl group; an aralkenyl group; an alkylaryl group; an alkoxysilylalkyl group; an arylphosphine group; or a heterocyclic group containing at least one of N, O and S atoms, or being unsubstituted or substituted with a substituent group to which two or more substituent groups of the above-exemplified substituent groups are linked.

As used herein, the alkyl group is a monovalent functional group derived from an alkane, and may be a straight-chain or a branched-chain. The number of carbon atoms of the straight chain alkyl group is not particularly limited, but is preferably 1 to 20. Also, the number of carbon atoms of the branched chain alkyl group is 3 to 20. Specific examples of the alkyl group include methyl, ethyl, propyl, n-propyl, isopropyl, butyl, n-butyl, isobutyl, tert-butyl, sec-butyl, 1-methyl-butyl, 1-ethyl-butyl, pentyl, n-pentyl, isopentyl, neopentyl, tert-pentyl, hexyl, n-hexyl, 1-methylpentyl, 2-methylpentyl, 4-methyl-2-pentyl, 3,3-dimethylbutyl, 2-ethylbutyl, heptyl, n-heptyl, 1-methylhexyl, octyl, n-octyl, tert-octyl, 1-methylheptyl, 2-ethylhexyl, 2-propylpentyl, n-nonyl, 2,2-dimethylheptyl, 1-ethyl-propyl, 1,1-dimethyl-propyl, isohexyl, 4-methylhexyl, 5-methylhexyl, 2,6-dimethylheptane-4-yl and the like, but are not limited thereto. The alkyl group may be substituted or unsubstituted, and when substituted, examples of the substituent are the same as described above.

As used herein, the cycloalkyl group is a monovalent functional group derived from a cycloalkane, and may be a monocyclic group or a polycyclic group and is not particularly limited, but the carbon number thereof is preferably 3 to 20. According to another embodiment, the carbon number of the cycloalkyl group is 3 to 10. Specific examples thereof include cyclopropyl, cyclobutyl, cyclopentyl, 3-methylcyclopentyl, 2,3-dimethylcyclopentyl, cyclohexyl, 3-methylcyclohexyl, 4-methylcyclohexyl, 2,3-dimethylcyclohexyl, 3,4,5-trimethylcyclohexyl, 4-tert-butylcyclohexyl, cycloheptyl, cyclooctyl, bicyclo[2,2,1]heptyl, and the like, but are not limited thereto. The cycloalkyl group may be substituted or unsubstituted, and when substituted, examples of the substituent are the same as described above.

As used herein, the aryl group is a monovalent functional group derived from an arene, and is not particularly limited, but the carbon number thereof is preferably 6 to 20, and it may be a monocyclic aryl group or a polycyclic aryl group. The aryl group may be a phenyl group, a biphenyl group, a terphenyl group or the like as the monocyclic aryl group, but is not limited thereto. The polycyclic aryl group includes a naphthyl group, an anthracenyl group, a phenanthryl group, a pyrenyl group, a perylenyl group, a chrysenyl group, a fluorenyl group, or the like, but is not limited thereto. The aryl group may be substituted or unsubstituted, and when substituted, examples of the substituent are the same as described above.

As used herein, the heteroaryl group includes one or more non-carbon atoms or heteroatoms, and specifically, the heteroatom may include one or more atoms selected from the group consisting of O, N, Se, S, and the like. The carbon number thereof is not particularly limited, but is preferably 4 to 20. The heteroaryl group may be a monocyclic group or a polycyclic group. Examples of the heteroaryl group include a thiophene group, a furanyl group, a pyrrole group, an imidazolyl group, a thiazoly group, an oxazoly group, an oxadiazoly group, a pyridyl group, a bipyridyl group, a pyrimidyl group, a triazinyl group, a triazolyl group, an acridyl group, a pyridazinyl group, a pyrazinyl group, a quinolinyl group, a quinazolinyl group, a quinoxalinyl group, a phthalazinyl group, a pyridopyrimidinyl group, a pyridopyrazinyl group, a pyrazinopyrazinyl group, an isoquinolinyl group, an indolyl group, a benzoxazolyl group, a benzoimidazolyl group, a benzothiazolyl group, a benzocarbazolyl group, a benzothiophene group, a dibenzothiophene group, a benzofuranyl group, a phenanthroline group, an isoxazolyl group, an oxadiazolyl group, a thiadiazolyl group, a benzothiazolyl group, a phenothiazinyl group, an aziridyl group, an azaindolyl group, an isoindolyl group, an indazolyl group, a purine group, a pteridine group, a beta-carbolyl group, a naphthyridine group, a tert-pyridyl group, a phenazinyl group, an imidazopyridyl group, a pyrropyridyl group, an azepine group, a pyrazolyl group, a dibenzofuranyl group, and the like, but are not limited thereto. The heteroaryl group may be substituted or unsubstituted, and when substituted, examples of the substituent are the same as described above.

As used herein, the alkylene group is a bivalent functional group derived from alkane, and the description of the alkyl group as defined above can be applied except that these are divalent functional groups. For example, the linear or branched alkylene group may include a methylene group, an ethylene group, a propylene group, an isobutylene group, a sec-butylene group, a tert-butylene group, a pentylene group, a hexylene group, and the like. The alkylene group can be substituted or unsubstituted, and when substituted, examples of the substituent are the same as described above.

As used herein, the arylene group is a divalent functional group derived from arene, and the description of the aryl group as defined above can be applied except that these are divalent functional groups. Examples thereof include a phenylene group, a biphenylene group, a terphenylene group, a naphthalene group, a fluorenyl group, a pyrenyl group, a phenanthrenyl group, a perylene group, a tetracenyl group, an anthracenyl group, and the like, but are not limited thereto. The arylene group may be substituted or unsubstituted, and when substituted, examples of the substituent are the same as described above.

As used herein, a cycloalkylene group is a divalent functional group derived from cycloalkane, and the description of the cycloalkyl group as defined above can be applied, except that it is a divalent functional group. The cycloalkylene group may be substituted or unsubstituted, and when substituted, examples of the substituent are as described above.

As used herein, the hetero arylene group has 2 to 20 carbon atoms, or 2 to 10 carbon atoms, or 6 to 20 carbon atoms. The description of the heteroaryl group as defined above can be applied except that the arylene group containing O, N or S as a heteroatom and is a divalent functional group. The hetero arylene group may be substituted or unsubstituted, and when substituted, examples of the substituent are as described above.

It should be understood that the terms "comprise," "include", "have", etc. are used herein to specify the presence of stated feature, region, integer, step, action, element and/or component, but do not preclude the presence or addition of one or more other feature, region, integer, step, action, element, component and/or group.

Further, the terms including ordinal numbers such as "a first", "a second", etc. are used only for the purpose of distinguishing one component from another component, and are not limited by the ordinal numbers. For instance, a first component may be referred to as a second component, or similarly, the second component may be referred to as the first component, without departing from the scope of the present invention.

### 1. Monomer Composition for Synthesizing Recycled Plastic

According to one embodiment of the present invention, there can be provided a monomer composition for synthesizing recycled plastic, comprising an aromatic diol compound, wherein a ratio of aromatic diol compound derivative impurities according to the following Equation 1 is 0.3% or less, wherein the melting point is 156.4°C or more, and wherein the monomer composition is recovered from a polycarbonate-based resin. Ratio of aromatic diol compound derivative impurities (%) = (Peak area of aromatic diol compound derivative measured by a liquid chromatography / Total peak area measured by a liquid chromatography) X 100.

The present inventors have found through experiments that although the monomer composition for synthesizing recycled plastics of the one embodiment was recovered through recycling by chemical decomposition of the polycarbonate-based resin, the aromatic diol compound, which is the main recovery target, can realize excellent color quality, and the aromatic diol compound can be secured in high purity, and completed the present invention.

In particular, together with the monomer composition (first composition) for synthesizing recycled plastic of the above embodiment, a monomer composition (second composition) for synthesizing recycled plastic comprising a carbonate-based compound such as a dialkyl carbonate, wherein the carbonate-based compound is recovered from a polycarbonate-based resin, can be respectively obtained at the same time by the method of preparing a monomer composition for synthesizing recycled plastic, which will be described later.

That is, the present invention may have technical features and advantages that the first composition including the aromatic diol compound can be obtained in high purity through recycling by chemical decomposition of a polycarbonate-based resin, and at the same time, the second composition including carbonate-based compound, which is a high value-added by-product, can also be obtained.

Specifically, the monomer composition for synthesizing recycled plastic according to the one embodiment is characterized by being recovered from a polycarbonate-based resin. That is, this means that as a result of proceeding the recovery from the polycarbonate-based resin in order to obtain the monomer composition for synthesizing recycled plastic according to the one embodiment, the monomer composition for synthesizing recycled plastics containing the aromatic diol compound is obtained together.

The polycarbonate-based resin is meant to include both a homopolymer and a copolymer containing a polycarbonate repeating unit, and collectively refers to a reaction product obtained through a polymerization reaction or a copolymerization reaction of a monomer containing an aromatic diol compound and a carbonate-based compound. When it contains one type of carbonate repeating unit obtained by using only one type of aromatic diol compound and one type of carbonate-based compound, a homopolymer can be synthesized. In addition, when one type of aromatic diol compound and two or more types of carbonate-based compounds are used as the monomer, or two or more types of aromatic diol compounds and one type of carbonate-based compound are used, or one or more types of other diols is used in addition to the one type of aromatic diol compound and the one types of carbonate-based compound to contain two or more types of carbonates, a copolymer can be synthesized. The homopolymer or copolymer can include all of low-molecular compounds, oligomers, and polymers depending on the molecular weight range.

Further, the monomer composition for synthesizing recycled plastics according the one embodiment may include an aromatic diol compound. Specific examples of the aromatic diol compound include bis(4-hydroxyphenyl)methane, bis(4-hydroxyphenyl)ether, bis(4-hydroxyphenyl)sulfone, bis(4-hydroxyphenyl)sulfoxide, bis(4-hydroxyphenyl)sulfide, bis(4-hydroxyphenyl)ketone, 1,1-bis(4-hydroxyphenyl)ethane, 2,2-bis(4-hydroxyphenyl)propane (bisphenol A), 2,2-bis(4-hydroxyphenyl)butane, 1,1-bis(4-hydroxyphenyl)cyclohexane (bisphenol Z), 2,2-bis(4-hydroxy-3,5-dibromophenyl)propane, 2,2-bis(4-hydroxy-3,5-dichlorophenyl)propane, 2,2-bis(4-hydroxy-3-bromophenyl)propane, 2,2-bis(4-hydroxy-3-chlorophenyl)propane, 2,2-bis(4-hydroxy-3-methylphenyl)propane, 2,2-bis(4-hydroxy-3,5-dimethylphenyl)propane, 1,1-bis(4-hydroxyphenyl)-1-phenylethane, or a mixture of two or more thereof, and the like. Preferably, the aromatic diol compound of the monomer composition for synthesizing recycled plastics of the one embodiment may be 2,2-bis(4-hydroxyphenyl)propane (bisphenol A).

The aromatic diol compound is characterized by being recovered from the polycarbonate-based resin used for recovering the monomer composition for synthesizing the recycled plastic. That is, this means that as a result of performing the recovery from the polycarbonate-based resin in order to obtain the monomer composition for synthesizing recycled plastic according to the one embodiment, an aromatic diol compound is also obtained together. Therefore, apart from the recovery from the polycarbonate-based resin in order to prepare the monomer composition for synthesizing recycled plastics according to the one embodiment, the case where a novel aromatic diol compound is added from the outside is not included in the category of aromatic diol compound of the present invention.

Specifically, "being recovered from the polycarbonate-based resin" means being obtained through a depolymerization reaction of the polycarbonate-based resin. The depolymerization reaction can be carried out under acidic, neutral or basic conditions, and particularly, the depolymerization reaction can be carried out under basic (alkaline) conditions. Particularly, the depolymerization reaction can be preferably carried out in the presence of an alcohol, as will be described later.

Meanwhile, the monomer composition for synthesizing recycled plastics of the one embodiment may have an APHA Color value of 60 or less, or 50 or less, or 40 or less, or 30 or less, or 20 or less, or 10 or less, or 0 or more, or 1 or more, or 0 to 60, or 0 to 50, or 0 to 40, or 0 to 30, or 0 to 20, or 0 to 10, or 1 to 60, or 1 to 50, or 1 to 40, or 1 to 30, or 1 to 20, or 1 to 10, which is measured according to ASTM D 1209.

Examples of the method for measuring the APHA Color of the monomer composition for synthesizing recycled plastics according to the one embodiment are not particularly limited, and for example, it can be measured according to the ASTM D 1209 test method using HunterLab UltraScan PRO Spectrophotometer device.

The APHA Color is a value obtained from a color comparison between water having no color and a PtCo solution having a yellow color. A value closer to 0 indicates colorless, and a value closer to 500 indicates yellow. Therefore, as the APHA Color of the monomer composition for synthesizing recycled plastic according to the one embodiment decreases to 60 or less, it is possible to realize transparent color properties in the synthesis of a polycarbonate-based resin using the same.

On the other hand, when the APHA Color of the monomer composition for synthesizing recycled plastic according to one embodiment excessively increases to more than 60 or the like, there is a limit in that it is difficult to synthesize a colorless and transparent polycarbonate-based resin due to yellowing.

Meanwhile, the monomer composition for synthesizing recycled plastic according to one embodiment may have an aromatic diol compound purity of 99.6% or more, or 99.7% or more, or 99.8% or more, or 99.9% or more, or 100% or less, or 99.6% to 100%, or 99.7% to 100%, or 99.8% to 100%, or 99.9% to 100%.

Examples of the method for measuring the purity of the aromatic diol compound of the monomer composition for synthesizing recycled plastics of the one embodiment are not particularly limited, and for example, ¹H NMR, ICP-MS analysis, HPLC analysis, UPLC analysis, etc. can be used without limitation. As for the specific methods, conditions, equipment, etc. of the NMR, ICP-MS, HPLC, and UPLC, various well-known contents can be applied without limitation.

As described above, in the monomer composition for synthesizing recycled plastics of the one embodiment, the purity of the aromatic diol compound, which is the main recovery target material, is remarkably increased to 99.6% or more, and other impurities are minimized, thereby capable of achieving excellent physical properties when synthesizing a polycarbonate-based resin using the same.

Meanwhile, the monomer composition for synthesizing recycled plastics of the one embodiment may have a melting point of 156.4°C or more, or 156.5°C or more, or 156.6°C or more, or 156.7°C or more, or 156.8°C or more, or 156.9°C or more, or 160°C or less, or 156.4°C to 160°C, or 156.5°C to 160°C, or 156.6°C to 160°C, or 156.7°C to 160°C, or 156.8°C to 160°C, or 156.9°C to 160°C. The reason why the melting point of the monomer composition for synthesizing recycled plastic is increased to 156.4°C or more appears to be due to the method for preparing the monomer composition for synthesizing recycled plastic, which will be described later.

Examples of the method for measuring the melting point of the monomer composition for synthesizing recycled plastic according to one embodiment is not particularly limited, and can be measured, for example, through a differential scanning calorimeter.

In this manner, the melting point of the monomer composition for synthesizing recycled plastic of one embodiment is increased to 156°C or more, and other impurities are minimized, thereby capable of realizing excellent physical properties in the synthesis of polycarbonate-based resins.

Meanwhile, the monomer composition for synthesizing recycled plastics may further include impurities other than the aromatic diol compound. The impurity refers to all materials except for the aromatic diol compound, which is the main recovery target material of the present invention.

The aromatic diol compound derivative may comprise one or more compounds selected from the group consisting of monoalkyl carbonate of bisphenol A, bisalkyl carbonate of bisphenol A, and bisphenol A dimer. The monoalkyl carbonate of bisphenol A is a product obtained by reacting the hydroxy group at one end of bisphenol A with dialkyl carbonate, and specific examples thereof are not particularly limited, but monoethyl carbonate of bisphenol A or monomethyl carbonate of bisphenol A may be mentioned as an example. The bisethyl carbonate of bisphenol A is a product obtained by reacting both hydroxyl groups at both ends of bisphenol A with dialkyl carbonate, and specific examples thereof are not particularly limited, but bisethyl carbonate of bisphenol A, or bismethyl carbonate of bisphenol A may be mentioned as an example. The bisphenol A dimer is a linear or cyclic product obtained by reacting bisphenol A with bisphenol A.

That is, the aromatic diol compound derivative may include one type of monoalkyl carbonate of bisphenol A, one type of bisalkyl carbonate of bisphenol A, one type of bisphenol A dimer, or a mixture of two types thereof.

By applying the method for preparing a monomer composition for synthesizing recycled plastic, which will be described later, the production of derivative impurities of the aromatic diol compound can be remarkably reduced.

Specifically, the monomer composition for synthesizing recycled plastic may have the ratio of the aromatic diol compound derivative impurities according to Equation 1 whose upper limit range is 0.3% or less, or 0.25% or less, or 0.2% or less, or 0.12% or less, or 0.1% or less, and whose lower limit range is 0.01% or more. A numerical range from the lower limit to the upper limit can also be satisfied by combining the upper limit range and the lower limit range. In one example of the numerical range of the lower limit to the upper limit, the ratio of the aromatic diol compound derivative impurities according to Equation 1 may be 0.01% to 0.3%. Ratio of aromatic diol compound derivative impurities (%) = (Peak area of aromatic diol compound derivative measured by a liquid chromatography / Total peak area measured by a liquid chromatography) X 100.

According to Equation 1, percentage (%), which is a unit of the ratio of the aromatic diol compound derivative impurities, is the peak area ratio measured by a liquid chromatography, and means area %.

In Equation 1, the peak area of the aromatic diol compound derivative measured by a liquid chromatography may be the total peak area of each of one or more aromatic diol compound derivatives. For example, when the aromatic diol compound derivative is a mixture of three types of monoalkyl carbonate of bisphenol A, bisalkyl carbonate of bisphenol A, and bisphenol A dimer, it means the total peak area of each of monoalkyl carbonate of bisphenol A, bisalkyl carbonate of bisphenol A, and bisphenol A dimer.

Examples of the method for measuring the weight ratio of the aromatic diol compound derivative impurities of the monomer composition for synthesizing recycled plastics according to the one embodiment is not particularly limited, and for example, high performance liquid chromatography (HPLC) analysis can be used. As for the specific methods, conditions, equipment, and the like of the HPLC, various well-known contents can be applied without limitation.

However, as an example, after the recycled bisphenol A monomer composition is dissolved in a methanol solvent at a concentration of 0.5 mg/mL under the conditions of normal pressure and 20~30°C, solid impurities in the solution are removed using a syringe filter (0.45µm), and then the weight ratio can be measured by UPLC (ultra performance liquid chromatography) via Waters HPLC system (e2695 separation module, 2998 PDA detector) using Capcell Pak C18 (4.6 mm ID x 50 mm L, 5 µm). More specifically, the weight ratio can be measured under the conditions of ① Column: Capcell Pak C18(4.6 mm ID x 50 mm L, 5µm), ② Column Temp: 40°C, ③ Injection volume: 10µℓ, ④ Flow: Mobile phase A-Acetonitrile/TFA=100/0.1%, B-Water/TFA=100/0.1%, Gradient elution - 0 min A:B=2:8, 10min A:B=100:0, volume=1ml/min (total=10min), and ⑤ Detector: 275 nm.

In this manner, in the monomer composition for synthesizing recycled plastic according to the one embodiment, the content of impurities of the aromatic diol compound derivative other than the aromatic diol compound, which are the main object materials to be recovered, is remarkably reduced, thereby capable of achieving excellent physical properties when synthesizing a polycarbonate-based resin using the same.

More particularly, a ratio of the monoalkyl carbonate of bisphenol A and the bisalkyl carbonate of bisphenol A according to the following Equation 2 may be 0.1% or less, or 0.05% or less, or 0% or more, or 0% to 0.1%, or 0% to 0.05%. Ratio of monoalkyl carbonate of bisphenol A and the bisalkyl carbonate of bisphenol A (%) = (Peak area of monoalkyl carbonate of bisphenol A and bisalkyl carbonate of bisphenol A measured by a liquid chromatography / Total peak area measured by a liquid chromatography) X 100.

Further, a ratio of the bisphenol A dimer according to the following Equation 3 may be 0.3 % or less, or 0.2% or less, or 0.13% or less, or 0.12% or less, or 0.1% or less, or 0.09% or less, or 0.01% or more, or 0.01% to 0.3%, or 0.01% to 0.2%, or 0.01% to 0.13%, or 0.01% to 0.12%, or 0.01% to 0.1%, or 0.01% to 0.09%. Ratio of the bisphenol A dimer (%) = (Peak area of bisphenol A dimer measured by a liquid chromatography / Total peak area measured by a liquid chromatography) X 100.

Meanwhile, in the monomer composition for synthesizing recycled plastic according to one embodiment, a carbonate-based compound is obtained as a by-product. The carbonate-based compound includes all compounds containing a carbonate functional group (-OCOO-), and may include carbonate compounds with a linear or cyclic structure. Further, the type of organic functional group bonded to the carbonate functional group in the carbonate-based compound is not particularly limited, and for example, it may include an alkyl group, a cycloalkyl group, an aryl group, a heteroaryl group, or a combination of two or more thereof.

More specifically, the carbonate-based compound may include dialkyl carbonate or diaryl carbonate. The dialkyl carbonate has a structure in which two alkyl groups are bonded to both ends of a carbonate functional group, and the two alkyl groups may be the same or different from each other. Specific examples of the dialkyl carbonate are not particularly limited, but include dialkyl carbonates having 3 to 15 carbon atoms, or 3 to 13 carbon atoms, such as dimethyl carbonate or diethyl carbonate.

Further, the diaryl carbonate has a structure in which two aryl groups are bonded to both ends of a carbonate functional group, and the two aryl groups may be the same or different from each other. Specific examples of the diaryl carbonate are not particularly limited, but include diaryl carbonates having 13 to 20 carbon atoms or 13 to 15 carbon atoms, such as diphenyl carbonate.

Since the main recovery target material in the monomer composition for synthesizing recycled plastics of the one embodiment is an aromatic diol compound, the carbonate-based compound can be separately separated and recovered as the by-product from the monomer composition for synthesizing recycled plastics of the one embodiment.

The monomer composition for synthesizing recycled plastics of the one embodiment can be used as a raw material for preparing various recycled plastics (e.g., polycarbonate (PC)) which will be described later.

The monomer composition for synthesizing recycled plastics according to the one embodiment may further include small amounts of other additives and solvents. Specific types of the additives or solvents are not particularly limited, and various materials widely used in the process of recovering the aromatic diol compound by a depolymerization of the polycarbonate-based resin can be applied without limitation.

The monomer composition for synthesizing recycled plastics of the one embodiment can be obtained by a method for preparing a monomer composition for synthesizing recycled plastics, which will be described later. That is, the monomer composition for synthesizing recycled plastics of one embodiment corresponds to the result obtained through various processes of filtration, purification, washing, and drying in order to secure only the aromatic diol compound, which is the main recovery target material, in high purity after the depolymerization reaction of the polycarbonate-based resin.

### 2. Method for preparing a monomer composition for synthesizing recycled plastic

### (1) First preparation method

According to another embodiment of the invention, there can be provided a method for preparing a monomer composition for synthesizing recycled plastic, the method comprising the steps of: subjecting a polycarbonate-based resin to a depolymerization reaction; separating a carbonate-based compound from the depolymerization reaction product; dissolving the depolymerization reaction product from which the carbonate-based compound has been separated in a solvent containing the separated carbonate-based compound; and recrystallizing the aromatic diol compound from the dissolved solution.

Specifically, the method for preparing a monomer composition for synthesizing recycled plastic according to the other embodiments may comprise a step of subjecting the polycarbonate-based resin to a depolymerization reaction.

The polycarbonate-based resin is meant to include both a homopolymer and a copolymer containing a polycarbonate repeating unit, and collectively refers to a reaction product obtained through a polymerization reaction or a copolymerization reaction of a monomer containing an aromatic diol compound and a carbonate-based compound. When it contains one carbonate repeating unit obtained by using only one type of aromatic diol compound and one type of carbonate-based compound, a homopolymer can be synthesized. In addition, when one type of aromatic diol compound and two or more types of carbonate-based compounds are used as the monomer, or two or more types of aromatic diol compounds and one type of carbonate-based compound are used, or one or more types of other diols is used in addition to the one type of aromatic diol compound and the one type of carbonate-based compound to contain two or more types of carbonates, a copolymer can be synthesized. The homopolymer or copolymer can include all of low-molecular compounds, oligomers, and polymers depending on the molecular weight range.

The polycarbonate-based resin can be applied regardless of various forms and types, such as a novel polycarbonate-based resin produced through synthesis, a recycled polycarbonate-based resin produced through a regeneration process, or polycarbonate-based resin waste.

However, if necessary, before subjecting the polycarbonate-based resin to a depolymerization reaction, a pretreatment step of the polycarbonate-based resin is carried out, thereby capable of increasing the efficiency of the process of recovering the aromatic diol compound and the carbonate-based compound. Examples of the pretreatment step may include washing, drying, grinding, glycol decomposition, and the like. The specific method of each pretreatment step is not limited, and various methods widely used in the process of recovering the aromatic diol compound and the carbonate-based compound from the polycarbonate-based resin can be applied without limitation.

During the depolymerization reaction of the polycarbonate-based resin, the depolymerization reaction may be carried out under acidic, neutral or basic conditions, and particularly, the depolymerization reaction may be carried out under basic (alkali) conditions. The type of the base is not particularly limited, and examples thereof include sodium hydroxide (NaOH) or potassium hydroxide (KOH). The base is a base catalyst acting as a catalyst, and has the economic advantages over organic catalysts, which are mainly used under mild conditions. More specifically, during the depolymerization reaction of the polycarbonate-based resin, the depolymerization reaction may proceed within a pH range of more than 8 and less than 12.

During the depolymerization reaction of the polycarbonate-based resin, the depolymerization reaction may be carried out by reacting a base in an amount of 0.5 moles or less, or 0.4 moles or less, or 0.3 moles or less, or 0.1 moles or more, or 0.2 moles or more, or 0.1 moles to 0.5 moles, or 0.1 moles to 0.4 moles, or 0.1 moles to 0.3 moles, or 0.2 moles to 0.5 moles, or 0.2 moles to 0.4 moles, or 0.2 moles to 0.3 moles relative to 1 mole of polycarbonate-based resin. When the polycarbonate-based resin is reacted with a base in an amount of more than 0.5 moles relative to 1 mole of the polycarbonate-based resin during depolymerization of the polycarbonate-based resin, there is a limit that impurities increase due to the effect of increasing the amount of alkali salt generated, so the purity of the target recovery material is reduced, and the economic efficiency of the catalytic reaction is reduced.

Further, the depolymerization reaction of the polycarbonate-based resin can be carried out in the presence of an alcohol. The present invention can stably obtain bisphenol A, which is a high-purity monomer, by decomposing a polycarbonate-based resin with an alcohol, and has the advantage that a carbonate-based compound such as dialkyl carbonate having a high added value can be further obtained as a reaction by-product.

The number of hydroxy groups in the alcohol is not particularly limited, but may include, for example, monohydric alcohol. The monohydric alcohol is a compound having one hydroxy group in the molecule.

Further, the type of monovalent organic functional group bonded to the hydroxy group in the monohydric alcohol is not particularly limited, but may include an organic functional group having 1 to 10 carbon atoms, or 1 to 6 carbon atoms, such as an alkyl group, a cycloalkyl group, an aryl group, a heteroaryl group, or a combination of two or more thereof.

The alcohol may be one type of single compound or a mixture of two or more types. Specific examples of the alcohol are not particularly limited, but examples thereof include ethanol, methanol, phenol, or mixtures thereof.

The content of the alcohol may be 5 moles to 15 moles, or 8 moles to 13 moles relative to 1 mole of the polycarbonate-based resin. Since the alcohol has good solubility in bisphenol A, alcohol within the above range should be essentially contained. When the content of the alcohol is excessively reduced to less than 5 moles relative to 1 mole of the polycarbonate-based resin, it is difficult to sufficiently progress the alcohol decomposition of polycarbonate-based resin. On the other hand, when the content of alcohol is excessively increased to more than 15 moles relative to 1 mole of the polycarbonate-based resin, the economics of the process can be reduced due to excessive use of alcohol.

The solvent in which the depolymerization reaction of the polycarbonate-based resin proceeds may include at least one organic solvent selected from the group consisting of tetrahydrofuran, toluene, methylene chloride, chloroform, dimethyl carbonate, ethylmethyl carbonate, diethyl carbonate, and dipropyl carbonate.

The organic solvent may include tetrahydrofuran, toluene, methylene chloride, chloroform, dimethyl carbonate, ethylmethyl carbonate, diethyl carbonate, dipropyl carbonate, or a mixture of two or more thereof.

More preferably, methylene chloride can be used as the organic solvent. When methylene chloride is used as the organic solvent, there is an advantage that the dissolution properties in polycarbonate can be improved and the reactivity can be enhanced.

The content of the organic solvent may be 16 moles to 20 moles, or 16 moles to 18 moles relative to 1 mole of the polycarbonate-based resin. In addition, the content of the organic solvent may be 1.5 moles to 2 moles relative to 1 mole of ethanol. By mixing the polycarbonate-based resin, alcohol, and an organic solvent within the above range, there is an advantage that the depolymerization reaction of the polymer can proceed at a desired level.

Meanwhile, the temperature at which the depolymerization reaction of the polycarbonate-based resin proceeds is not particularly limited, but for example, the reaction may proceed at a temperature of 20°C to 100°C, or 50°C to 70°C. In addition, the depolymerization reaction of the polycarbonate-based resin may proceed for 1 hour to 30 hours, or 4 hours to 6 hours.

Specifically, the conditions are mild process conditions relative to the conventional pressurizing/high temperature process, and by performing stirring under the above conditions, the process can be performed in a mild process as compared to the pressurizing/high temperature process. In particular, when stirring at 50°C to 70°C for 4 to 6 hours, there is an advantage of obtaining the most efficient results in terms of reproducibility and stability.

That is, according to the present invention, as the type and mixing amount of the mixed solvent and the type and content of the base catalyst is adjusted without using an organic catalyst, there is the advantage that a high-purity aromatic diol compound (e.g., bisphenol A) can be obtained under mild conditions without using a pressurizing/high temperature process, and alcohol is used as a reactant and thus, a carbonate-based compound such as dialkyl carbonate can be obtained as by-products.

Meanwhile, during the depolymerization reaction of the polycarbonate-based resin, an antioxidant can be added to the reaction solution. As the antioxidant is added, the aromatic diol compound recovered through recycling by chemical decomposition of the polycarbonate-based resin can satisfy a color level comparable to reagents commercially sold or used for a PC polymerization.

Specific examples of the antioxidant are not particularly limited, and various antioxidants that have been widely used in the prior art can be applied without limitation. However, one example thereof include sodium hyposulfite, sodium sulfite, erythorbic acid, dibutylhydroxytoluene, butylhydroxyanisole, α-tocopherol, tocopherol acetate, L-ascorbic acid and salts thereof, L-ascorbic acid palmitate, L-ascorbic acid stearate, triamyl gallate, propyl gallate or ethylenediamine tetraacetic acid disodium salt(EDTA), sodium pyrophosphate, sodium metaphosphate, or a mixture of two or more thereof.

The specific addition amount of the antioxidant is also not particularly limited, but as an example, the antioxidant can be added at a level that does not affect the physical properties of the monomer composition for synthesizing recycled plastic within the range of 0.1% by weight to 5 % by weight, or 0.1 % by weight to 1 % by weight based on the total weight of the reaction solution.

In addition, during the depolymerization reaction of the polycarbonate-based resin, the depolymerization can be carried out under a nitrogen atmosphere.

More specifically, the step of subjecting a polycarbonate-based resin to a depolymerization reaction may comprise a first step of dissolving the polycarbonate-based resin in an organic solvent; and a second step of adding a catalyst solution containing an alcohol, a base and an antioxidant. In the first and second steps, the details of the alcohol, organic solvent, base, antioxidant and polycarbonate-based resin are the same as described above.

Meanwhile, the method for preparing the monomer composition for synthesizing recycled plastics according to the other embodiment may further include a step of adding an acid so that the pH of the depolymerization reaction product becomes 2 to 8. A strong acid can be used as the acid, and examples thereof include hydrochloric acid (HCl).

In the step of adding an acid so that the pH of the depolymerization reaction product becomes 2 to 8, the salt of the aromatic diol compound included in the depolymerization reaction product may be converted into the aromatic diol compound. As the depolymerization reaction proceeds under basic conditions, the resulting aromatic diol compound exists in the form of a salt upon reaction with a base, and thus has hydrophilicity. Thereby, by adding an acid, the salt of the aromatic diol compound contained in the depolymerization reaction product can be converted into the aromatic diol compound, thereby inducing to have hydrophobicity.

Thus, a layer divided into a water layer containing impurities and an organic solvent layer containing an aromatic diol compound and a carbonate-based compound can be formed in the step of adding water to remove impurities after adding the acid, which will be described later. Since the aromatic diol compound and the carbonate-based compound have hydrophobicity, they may be included in an organic solvent layer among water layer and an organic solvent layer, and various water-soluble impurities can be included in the water layer. Thereby, the main products, i.e., aromatic diol compounds and impurities, can be easily separated only by a simple step of changing the pH.

Meanwhile, the method for preparing the monomer composition for synthesizing recycled plastics according to the other embodiment may further include a step of adding water in the step of adding an acid so that the pH of the depolymerization product is 2 to 8. Thereby, a layer divided into a water layer containing impurities and an organic solvent layer containing an aromatic diol compound and a carbonate-based compound can be formed in the step of removing impurities which will be described later.

The order of the step of adding the acid and the step of adding water is not particularly limited, and addition of water after addition of acid, addition of acid after addition of water, or simultaneous addition of water and acid are possible.

Meanwhile, the method for preparing the monomer composition for synthesizing recycled plastics according to the other embodiment may include a step of removing impurities after the addition of acid. Therefore, the impurities are materials having hydrophilicity, and examples thereof may include a salt compound, an ionic compound, an acid compound, and the like.

As described above, after the step of adding an acid so that the pH of the depolymerization reaction product is 2 to 8, as the step of removing impurities from the depolymerization reaction product after addition of the acid progresses, the depolymerization reaction product forms a layer divided into a water layer containing impurities, and an organic solvent layer containing an aromatic diol compound and a carbonate-based compound, so that the water layer containing impurities can be separated and removed.

In the step of removing impurities from the depolymerization reaction product after addition of the acid, the water layer can be separated from the organic layer to remove impurities included in the water layer. Specific separation conditions for separating the water layer from the organic layer are not particularly limited. As for the specific separation devices and methods, various well-known purification techniques can be applied without limitation. However, as an example, a drain device can be used.

Meanwhile, the method for preparing the monomer composition for synthesizing recycled plastics according to the other embodiment may include a step of separating the carbonate-based compound from the depolymerization reaction product.

The carbonate-based compound includes all compounds containing a carbonate functional group(-OCOO-), and may include carbonate compounds with a linear or cyclic structure. In addition, the type of organic functional group bonded to the carbonate functional group in the carbonate-based compound is not particularly limited, but may include, for example, an alkyl group, a cycloalkyl group, an aryl group, a heteroaryl group, or a combination of two or more thereof.

More specifically, the carbonate-based compound may include a dialkyl carbonate or a diaryl carbonate. The dialkyl carbonate has a structure in which two alkyl groups are bonded to both ends of a carbonate functional group, and the two alkyl groups may be the same or different from each other. Specific examples of the dialkyl carbonate are not particularly limited, but may include a dialkyl carbonate having 3 to 15 carbon atoms, or 3 to 13 carbon atoms, such as dimethyl carbonate or diethyl carbonate.

Further, the diaryl carbonate has a structure in which two aryl groups are bonded to both ends of a carbonate functional group, and the two aryl groups may be the same or different from each other. Specific examples of the diaryl carbonate are not particularly limited, but may include a diaryl carbonate having 13 to 20 carbon atoms, or 13 to 15 carbon atoms, such as diphenyl carbonate.

The step of separating the carbonate-based compound from the depolymerization reaction product may include a step of evaporating the depolymerization reaction product. Examples of the evaporation conditions are not particularly limited, but as a specific example, the product of the depolymerization reaction of the polycarbonate-based resin can be evaporated using an evaporator (e.g., a thin film evaporator).

The step of separating the carbonate-based compound from the depolymerization reaction product may include a distillation step of the depolymerization product. Examples of the distillation conditions are not particularly limited, but in a specific example, the product of the depolymerization reaction of the polycarbonate-based resin is pressurized under a pressure of 200 mbar to 300 mbar and a temperature of 20°C to 30°C, and then decompressed under a pressure of 10 mbar to 50 mbar and a temperature of 20°C to 30°C and subjected to a low-temperature distillation.

The order of proceeding the evaporation and distillation steps is not particularly limited, and the evaporation step and distillation step can be performed simultaneously or sequentially. That is, the evaporation step and the distillation step can be carried out simultaneously, the distillation step can be carried out after the evaporation step, or the evaporation step can be carried out after the distillation step.

The separated carbonate-based compound can be recycled without a separate purification process, or can be recycled through separation and purification such as conventional extraction, adsorption, and drying, if necessary. Specific purification conditions are not particularly limited. As for the specific purification devices and methods, various well-known purification techniques can be applied without limitation.

Meanwhile, the method for preparing the monomer composition for synthesizing recycled plastics according to the other embodiment may include a step of dissolving the depolymerization reaction product from which the carbonate-based compound has been separated in a solvent containing the separated carbonate-based compound. Through the step of dissolving the depolymerization reaction product from which the carbonate-based compound has been separated in a solvent containing the separated carbonate-based compound, the aromatic diol compound crystals contained in the depolymerization product from which the carbonate-based compound has been separated may be redissolved in the solvent.

Through such steps, the solubility of the aromatic diol compound or its salt contained in the depolymerization reaction product is increased, and thus, crystals, or impurities interposed between crystals can be dissolved with a solvent to the maximum, and further, since the dissolved aromatic diol compound has poor solubility relative to impurities, it can be easily precipitated into aromatic diol compound crystals through the difference in solubility when the temperature is lowered subsequently.

Conventionally, there was a limitation to the reduction in process efficiency due to the use of a separate fresh ethanol solution, but in the present invention, the process efficiency can be remarkably improved by recycling the carbonate-based compound separated in the previous step and applying it as a re-dissolution solvent.

The solvent may contain a separated carbonate-based compound. The separated carbonate-based compound is a product obtained in the step of separating the carbonate-based compound from the depolymerization reaction product, and the solvent may consist only of the separated carbonate-based compound, or may be one in which the separated carbonate-based compound occupies most of the main component and some impurities are mixed therein.

The step of dissolving the depolymerization reaction product from which the carbonate-based compound has been separated in a solvent containing the separated carbonate-based compound may be carried out at a temperature of 0°C to 200°C, or 50°C to 150°C, or 50°C to 120°C, or 50°C to 100°C.

The addition amount of the solvent containing the carbonate-based compound may be 10 parts by weight or more, or 40 parts by weight or more, or 90 parts by weight or more, or 100 parts by weight or more, or 200 parts by weight or less, or 10 parts by weight to 200 parts by weight, or 40 parts by weight to 200 parts by weight, or 90 parts by weight to 200 parts by weight, or 100 parts by weight to 200 parts by weight based on 100 parts by weight of the aromatic diol compound contained in the depolymerization reaction product from which the carbonate-based compound has been separated. If the solvent containing the carbonate-based compound is used in an excessively small amount, the temperature for dissolving the aromatic diol compound contained in the depolymerization reaction product from which the carbonate-based compound has been separated becomes too high, which leads to deterioration in the process efficiency. As the temperature difference between the dissolving temperature and the cooling temperature increases, it is not easy to remove impurities through recrystallization. In addition, since the solid concentration after recrystallization is too high, transportation or solid-liquid separation becomes very difficult, and therefore it may be not easy to remove impurities.

On the other hand, if a solvent containing a carbonate-based compound is used in excessive amounts, the solubility of the aromatic diol compound contained in the depolymerization reaction product from which the carbonate-based compound has been separated becomes too high, which leads to decrease in the yield of the aromatic diol compound recovered after recrystallization, and the process efficiency can be reduced due to the use of large amounts of solvent.

Meanwhile, the method for preparing a monomer composition for synthesizing recycled plastic according to another embodiment may include a step of recrystallizing an aromatic diol compound from the dissolved solution. The aromatic diol compound can be obtained with high purity through the recrystallization step.

The concentration of the aromatic diol compound contained in the solution may be 90% by weight or less, or 70% by weight or less, or 60% by weight or less, or 50% by weight or less, or 40% by weight or more, or 40% by weigh to 90% by weight, or 40% by weight to 70% by weight, or 40% by weigh to 60% by weight, or 40% by weigh to 70% by weight. The concentration of the aromatic diol compound contained in the solution means the mass percentage of the aromatic diol compound relative to the mass of the entire solution.

If the concentration of the aromatic diol compound contained in the solution is excessively increased, it is not easy to remove impurities through recrystallization. Meanwhile, if the concentration of the aromatic diol compound contained in the solution decreases excessively, the yield of the aromatic diol compound recovered after recrystallization may be decreased.

In the step of recrystallizing the aromatic diol compound from the dissolved solution, specific examples of the recrystallization method are not particularly limited, and various methods such as cooling, evaporation, and flash cooling, which are widely known as conventional recrystallization methods, can be applied without limitation.

However, as an example, the step of recrystallizing the aromatic diol compound from the dissolved solution may include a step of cooling the dissolved solution. The step of cooling the dissolved solution may be carried out at a temperature of 0°C to 40°C, or 5°C to 40°C, or 5°C to 30°C, or 5°C to 20°C. The temperature difference between the step of dissolving the depolymerization reaction product from which the carbonate-based compound has been separated in a solvent containing the separated carbonate-based compound; and the step of recrystallizing the aromatic diol compound from the dissolved solution, may be 40°C to 100°C, or 40°C to 80°C, or 50°C to 70°C.

The step of dissolving the depolymerization reaction product from which the carbonate-based compound has been separated in a solvent containing the separated carbonate-based compound; and the step of recrystallizing the aromatic diol compound from the dissolved solution, may be repeated two or more times. That is, the step of dissolving the depolymerization reaction product from which the carbonate-based compound has been separated in a solvent containing the separated carbonate-based compound; and the step of recrystallizing the aromatic diol compound from the dissolved solution, can proceed again one or more times. Through this, the aromatic diol compound to be recovered can achieve excellent color quality.

On the other hand, the method may further comprise a step of adding an adsorbent to the separated depolymerization reaction product to perform an adsorption purification and then removing the adsorbent, between the step of separating a carbonate-based compound from the depolymerization reaction product; and the step of dissolving the depolymerization reaction product from which the carbonate-based compound has been separated in a solvent containing the separated carbonate-based compound.

Further, in the step of adding an adsorbent to the depolymerization reaction product from which the carbonate-based compound has been separated, to perform an adsorption purification and then removing the adsorbent, an adsorbent can be brought into contact with the depolymerization reaction product.

Examples of the adsorbent that can be used include activated carbon, charcoal, or a mixture thereof. The activated carbon is a black carbon material having micropores produced by subjecting a raw material to a carbonization process at about 500°C and an activated carbon process at about 900°C, and examples thereof are not particularly limited, but for example, various activated carbons such as plant-based, coal-based, petroleum-based, waste-based activated carbons can be applied without limitation depending on the type of raw material.

In more specific examples, the plant-based activated carbon may include coconut activated carbon, wood activated carbon, and sawdust activated carbon. Further, the coal-based activated carbon may include lignite activated carbon, bituminous coal activated carbon, and anthracite activated carbon. Further, the petroleum-based activated carbon may include petroleum coke activated carbon and oil carbon activated carbon. Further, the waste activated carbon may include synthetic resin activated carbon and pulp activated carbon.

The adsorbent may include at least one activated carbon selected from the group consisting of plant-based activated carbon, coal-based activated carbon, petroleum-based activated carbon, and waste-based activated carbon. That is, the adsorbent may include plant-based activated carbon, coal-based activated carbon, petroleum-based activated carbon, waste-based activated carbon, or a mixture of two or more thereof.

More specifically, the adsorbent may include at least one activated carbon selected from the group consisting of coconut activated carbon, lignite activated carbon, anthracite activated carbon, and bituminous coal activated carbon. That is, the adsorbent may include coconut activated carbon, lignite activated carbon, anthracite activated carbon, bituminous coal activated carbon, or a mixture of two or more thereof.

The adsorption purification conditions by the adsorbent are not particularly limited, and various well-known adsorption purification conditions can be used without limitation. However, in one example, the addition amount of the adsorbent may be 40% to 60% by weight relative to the polycarbonate-based resin, and the adsorption time may be 1 hour to 5 hours, and the adsorption method may be a stirring adsorption or an adsorption tower for Lab.

If necessary, the method may further include a step of adding a solvent to the depolymerization product from which the carbonate-based compound has been separated, prior to the step of adding an adsorbent to the depolymerization reaction product from which the carbonate-based compound has been separated to perform an adsorption purification and then removing the adsorbent. Examples of the solvent include ethanol, and the ethanol may be added in a ratio of 1 mole to 20 moles, or 10 moles to 20 moles, or 15 moles to 20 moles relative to 1 mole of the polycarbonate-based resin. Aromatic diol compound crystals contained in the depolymerization reaction product from which the carbonate-based compound has been separated can be redissolved in a solvent through the step of adding a solvent to the depolymerization reaction product in which the carbonate-based compound has been separated.

Meanwhile, after the step of adding a solvent to the depolymerization product from which the carbonate-based compound has been separated, the method may further comprise a step of recrystallizing the depolymerization reaction product from which the carbonate-based compound has been separated. Examples of the evaporation conditions are not particularly limited, but as a specific example, evaporation can be performed using an evaporator (e.g., a thin film evaporator). After proceeding the evaporation step, a step of dissolving the depolymerization reaction product from which the carbonate-based compound has been separated in a solvent containing the separated carbonate-based compound can be proceeded.

Meanwhile, after the step of adding a solvent to the depolymerization reaction product from which the carbonate-based compound has been separated, the method may further include a step of recrystallizing the depolymerization reaction product from which the carbonate-based compound has been separated. In the recrystallization step of the depolymerization reaction product from which the carbonate-based compound has been separated, various impurities contained in the depolymerization reaction product from which the carbonate-based compound has been separated can be sufficiently removed, thereby ensuring a high-purity aromatic diol compound.

Specifically, the recrystallization step may include a step of adding water to the depolymerization reaction product from which the carbonate-based compound has been separated to perform recrystallization. Through the step of adding water to the depolymerization reaction product from which the carbonate-based compound has been separated to perform recrystallization, the solubility of the aromatic diol compound or its salt contained in the depolymerization reaction product is increased, and thus, crystals, or impurities interposed between crystals can be dissolved with a solvent to the maximum, and further, since the dissolved aromatic diol compound has poor solubility relative to impurities, it can be easily precipitated into aromatic diol compound crystals through the difference in solubility when the temperature is lowered subsequently.

More specifically, in the step of adding water to the depolymerization reaction product from which the carbonate-based compound has been separated to perform recrystallization, 200 moles to 400 moles, or 250 moles to 350 moles of water can be used with respect to 1 mole of the polycarbonate-based resin. When the water is used in an excessively small amount, the temperature for dissolving the aromatic diol compound contained in the depolymerization reaction product from which the carbonate-based compound has been separated becomes too high, which thus deteriorates in the process efficiency, and it is difficult to remove impurities through recrystallization. On the other hand, when water is used in an excessively large amount, the solubility of the aromatic diol compound contained in the depolymerization reaction product from which the carbonate-based compound has been separated becomes too high, and thus, the yield of the aromatic diol compound recovered after recrystallization is reduced, and the process efficiency can be reduced due to the use of large amounts of solvent.

After proceeding the recrystallization step of the depolymerization reaction product from which the carbonate-based compound has been separated, a step of dissolving the depolymerization reaction product from which the carbonate-based compound has been separated in a solvent containing the separated carbonate-based compound can be proceeded. However, if necessary, after proceeding the recrystallization step of the depolymerization reaction product from which the carbonate-based compound has been separated, a step of removing remaining impurities through filtration or adsorption may be further carried out.

Further, the method may further comprise a step of adding an adsorbent to the depolymerization reaction product from which the carbonate-based compound has been separated, to perform an adsorption purification and then removing the adsorbent, between the step of dissolving the depolymerization reaction product from which the carbonate-based compound has been separated in a solvent containing the separated carbonate-based compound; and the step of recrystallizing the aromatic diol compound from the dissolved solution. The details of the step of adding an adsorbent to the depolymerization reaction product from which the carbonate-based compound has been separated, to perform an adsorption purification and then removing the adsorbent are the same as described above.

Meanwhile, the method for preparing a monomer composition for synthesizing recycled plastic according to the other embodiment may further comprise a step of washing the depolymerization reaction product from which the carbonate-based compound has been separated, between the step of separating a carbonate-based compound from the depolymerization reaction product; and the dissolving the depolymerization reaction product from which the carbonate-based compound has been separated in a solvent containing the separated carbonate-based compound.

In the step of washing the depolymerization reaction product from which the carbonate-based compound has been separated, the depolymerization reaction product from which the carbonate-based compound has been separated may contain an aromatic diol compound. However, since various impurities remain during the recovery process for obtaining the aromatic diol compound, washing can proceed in order to sufficiently remove these impurities and secure a high-purity aromatic diol compound.

Specifically, the washing step may include a step of washing with a solvent at a temperature of 10°C or more and 30°C or less, or 20°C or more and 30°C or less. The temperature condition means the temperature inside a washing container at which washing with a solvent is proceeded. In order to maintain a high temperature deviating from a room temperature, various heating devices can be applied without limitation.

In the washing step, after proceeding the step of washing with a solvent at a temperature of 10°C or more and 30°C or less, a step of removing a solvent remaining through filtration may be further carried out.

The solvent used in the washing step may include one of water, alcohol, and an organic solvent. As the organic solvent, tetrahydrofuran, toluene, methylene chloride, chloroform, dimethyl carbonate, ethylmethyl carbonate, diethyl carbonate, dipropyl carbonate, or a mixture of two or more thereof can be used.

The solvent used in the washing step can be used in a weight ratio of 1 part by weight or more and 30 parts by weight or less, or 1 part by weight or more and 10 parts by weight or less based on 1 part by weight of the polycarbonate-based resin used in the depolymerization reaction.

More specifically, the solvent in the step of washing with a solvent at a temperature of 10°C or more and 30°C or less may be an organic solvent. Preferably, methylene chloride can be used as the organic solvent. At this time, the organic solvent can be used in an amount of 1 part by weight or more and 10 parts by weight or less based on 1 part by weight of the polycarbonate-based resin.

Meanwhile, if necessary, after proceeding the recrystallization step of the depolymerization reaction product from which the carbonate precursor has been separated, a step of removing impurities remaining through filtration or adsorption can be further carried out.

In addition, if necessary, after the recrystallization step, the method may further include a drying step. The remaining solvent can be removed through the drying, and the specific drying conditions are not particularly limited, but for example, the drying can be performed at a temperature of 10°C to 100°C, or 50°C to 100°C. As for the specific drying equipment and method used in the drying, various well-known drying techniques can be applied without limitation.

### (2) Second preparing method

Meanwhile, according to another embodiment of the invention, there can be provided a method for preparing a monomer composition for synthesizing recycled plastic, the method comprising the steps of: subjecting a polycarbonate-based resin to a depolymerization reaction; dissolving the depolymerization reaction product in a solvent containing a carbonate-based compound; and recrystallizing the aromatic diol compound from the dissolved solution.

For convenience, the method for preparing the monomer composition for synthesizing recycled plastic according to the other embodiments is referred to as the first preparation method, and the method for preparing the monomer composition for synthesizing recycled plastics according to yet another embodiment is referred to as the second preparation method.

The second preparation method may include the step of subjecting the polycarbonate-based resin to a depolymerization reaction, and the details of the step of subjecting the polycarbonate-based resin to a depolymerization reaction may include those described above in the first preparation method as they are.

Meanwhile, the second preparation method may further include, after the step of subjecting the polycarbonate-based resin to a depolymerization reaction, a step of adding an acid so that the pH of the depolymerization reaction product becomes 2 to 8, and the details of the step of adding an acid so that the pH of the depolymerization reaction product becomes 2 to 8 can include those described above in the first preparation method as they are.

The second preparation method can include a step of removing impurities after addition of an acid. The details of the step of removing impurities can include those described above in the first preparation method as they are.

The second preparation method can include a step of dissolving the depolymerization reaction product in a solvent containing a carbonate compound. The details of the depolymerization reaction product and carbonate-based compound may include those described above in the first preparation method as they are. The aromatic diol compound crystals contained in the depolymerization reaction product can be redissolved in the solvent through the step of adding a solvent containing a carbonate compound to the depolymerization reaction product.

Through such steps, the solubility of the aromatic diol compound or its salt contained in the depolymerization reaction product is increased, and thus, crystals, or impurities interposed between crystals can be dissolved with a solvent to the maximum, and further, since the dissolved aromatic diol compound has poor solubility relative to impurities, it can be easily precipitated into aromatic diol compound crystals through the difference in solubility when the temperature is lowered subsequently.

The solvent may contain a carbonate-based compound. The solvent may consist only of the carbonate-based compound, or may be one in which the carbonate-based compound occupies most of the main component and some impurities are mixed therein. The method for preparing the carbonate-based compound is not particularly limited, and all carbonate-based compounds obtained by conventionally known methods for preparing carbonate compounds can be applied without limitation.

In one example, it may be obtained from a depolymerization reaction of a polycarbonate-based resin, or may be newly synthesized industrially, or may be obtained by performing a depolymerization reaction and a new synthesis in parallel.

The step of dissolving the depolymerization product in a solvent containing the carbonate-based compound may be carried out at a temperature of 0°C to 200°C, or 50°C to 150°C, or 50°C to 120°C, or 50°C to 100°C.

The addition amount of the solvent containing the carbonate-based compound may be 10 parts by weight or more, or 40 parts by weight or more, or 90 parts by weight or more, or 100 parts by weight or more, or 200 parts by weight or less, or 10 parts by weight to 200 parts by weight, or 40 parts by weight to 200 parts by weight, or 90 parts by weight to 200 parts by weight, or 100 parts by weight to 200 parts by weight based on 100 parts by weight of the aromatic diol compound contained in the depolymerization reaction product. If the solvent containing the carbonate-based compound is used in an excessively small amount, the temperature for dissolving the aromatic diol compound contained in the depolymerization reaction product becomes too high, which leads to deterioration in the process efficiency. As the temperature difference between the dissolving temperature and the cooling temperature increases, it is not easy to remove impurities through recrystallization. In addition, since the solid concentration after recrystallization is too high, transportation or solid-liquid separation becomes very difficult, and therefore it may be not easy to remove impurities.

On the other hand, if a solvent containing a carbonate-based compound is used in excessive amounts, the solubility of the aromatic diol compound contained in the depolymerization reaction product becomes too high, which leads to decrease in the yield of the aromatic diol compound recovered after recrystallization, and the process efficiency can be reduced due to the use of large amounts of solvent.

Meanwhile, the second preparation method may include a step of recrystallizing the aromatic diol compound from the dissolved solution, and the details of the step of recrystallizing the aromatic diol compound from the dissolved solution may include those described above in the first preparation method as they are.

Meanwhile, in the second preparation method, a step of dissolving the depolymerization reaction product in a solvent containing a carbonate-based compound; and a step of recrystallizing the aromatic diol compound from the dissolved solution, may be repeated two or more times. That is, after the step of dissolving the depolymerization reaction product in a solvent containing the carbonate-based compound; and the step of recrystallizing the aromatic diol compound from the dissolved solution, are carried out once, the step of dissolving the depolymerization reaction product in a solvent containing a carbonate compound and the step of recrystallizing the aromatic diol compound in the dissolved solution can be carried out once or more again. Through this, the aromatic diol compound to be recovered can achieve excellent color quality.

Meanwhile, the second preparation method may further comprise a step of adding an adsorbent to the depolymerization reaction product to perform an adsorption purification and then removing the adsorbent, between the step of subjecting a polycarbonate-based resin to a depolymerization reaction; and the step of dissolving the depolymerization reaction product in a solvent containing the carbonate-based compound. The details of the step of adding an adsorbent to the separated depolymerization reaction product to perform an adsorption purification and then removing the adsorbent may include those described above in the first preparation method as they are.

Meanwhile, the second preparation method may further comprise a step of adding an adsorbent to the depolymerization reaction product to perform an adsorption purification and then removing the adsorbent, between the step of dissolving the depolymerization reaction product in a solvent containing the carbonate-based compound; and the step of recrystallizing the aromatic diol compound from the dissolved solution. The details of the step of adding an adsorbent to the separated depolymerization reaction product to perform an adsorption purification and then removing the adsorbent may include those described above in the first preparation method as they are.

Meanwhile, the second preparation method may further comprise a step of washing the depolymerization reaction product, between the step of subjecting a polycarbonate-based resin to a depolymerization reaction; and the step of dissolving the depolymerization reaction product in a solvent containing the carbonate-based compound. The details of the step of washing the depolymerization reaction product may include those described above in the first preparation method as they are.

### 3. Recycled Plastic

According to yet another embodiment of the invention, a recycled plastic comprising a reaction product of the monomer composition for synthesizing recycled plastic according to the one embodiment and a comonomer can be provided.

The details of the monomer composition for synthesizing recycled plastic according to the one embodiment include all the contents described above in the one embodiment.

Examples corresponding to the recycled plastic are not particularly limited, and various plastics synthesized from aromatic diol compounds such as bisphenol A and a carbonate-based compound such as dimethyl carbonate, diethyl carbonate, or ethylmethyl carbonate as a monomer can be applied without limitation, and a more specific example may be a polycarbonate-based resin.

The polycarbonate-based resin is meant to include both a homopolymer and a copolymer containing a polycarbonate repeating unit, and collectively refers to a reaction product obtained through a polymerization reaction or a copolymerization reaction of a monomer containing an aromatic diol compound and a carbonate-based compound. When it contains one type of carbonate repeating unit obtained by using only one type of aromatic diol compound and one type of carbonate-based compound, a homopolymer can be synthesized. In addition, when one type of aromatic diol compound and two or more types of carbonate-based compounds are used as the monomer, or two or more types of aromatic diol compounds and one type of carbonate-based compound are used, or one or more types of other diols is used in addition to the one type of aromatic diol compound and the one type of carbonate-based compound to contain two or more carbonates, a copolymer can be synthesized. The homopolymer or copolymer can include all of low-molecular compounds, oligomers, and polymers depending on the molecular weight range.

More specifically, in the recycled plastic containing the reaction product of the monomer composition for synthesizing the recycled plastic and the comonomer according to the one embodiment, a carbonate-based compound can be used as the comonomer. Specific examples of the carbonate-based compound include phosgene, triphosgene, diphosgene, bromophosgene, dimethyl carbonate, diethyl carbonate, dibutyl carbonate, dicyclohexyl carbonate, diphenyl carbonate, ditolyl carbonate, bis(chlorophenyl)carbonate, m-cresyl carbonate, dinaphthyl carbonate, bis(diphenyl) carbonate or bishaloformate.

Examples of the reaction process of the monomer composition for synthesizing recycled plastic and the comonomer that synthesizes the polycarbonate-based resin are not particularly limited, and various well-known methods for preparing polycarbonate can be applied without limitation.

However, in one example of the method for preparing a polycarbonate, a method for preparing a polycarbonate comprising a step of polymerizing a composition containing a monomer composition for synthesizing recycled plastic and a comonomer can be used. At this time, the polymerization can be carried out by interfacial polymerization, and during interfacial polymerization, polymerization reaction is possible at normal pressure and low temperature, and the molecular weight is easy to control.

The polymerization temperature may be 0°C to 40°C, and the reaction time may be 10 minutes to 5 hours. In addition, the pH during the reaction may be maintained at 9 or more or 11 or more.

The solvent that can be used for the polymerization is not particularly limited as long as it is a solvent used for polymerization of polycarbonate in the art, and as an example, halogenated hydrocarbons such as methylene chloride and chlorobenzene can be used.

Moreover, the polymerization can be carried out in the presence of an acid binder. As the acid binder, an alkali metal hydroxide such as sodium hydroxide or potassium hydroxide, or an amine compound such as pyridine can be used.

Further, in order to adjust the molecular weight of the polycarbonate during the polymerization, polymerization can be carried out in the presence of a molecular weight modifier. An alkylphenol having 1 to 20 carbon atoms may be used as the molecular weight modifier, and specific examples thereof include p-tert-butylphenol, p-cumylphenol, decylphenol, dodecylphenol, tetradecylphenol, hexadecylphenol, octadecylphenol, eicosylphenol, docosylphenol or triacontylphenol. The molecular weight modifier can be added before, during or after the initiation of polymerization. The molecular weight modifier may be used in an amount of 0.01 to 10 parts by weight, or 0.1 to 6 parts by weight, based on 100 parts by weight of the aromatic diol compound, and a desired molecular weight can be obtained within this range.

In addition, in order to promote the polymerization reaction, a reaction accelerator such as a tertiary amine compound, a quaternary ammonium compound, or a quaternary phosphonium compound, including triethylamine, tetra-n-butylammonium bromide, or tetra-n-butylphosphonium bromide can be further used.

### 4. Molded Product

According to yet another embodiment of the invention, a molded article comprising the recycled plastic according to the other embodiment can be provided. The details of the recycled plastic includes all the contents described above in the other embodiments.

The molded article can be obtained by applying the recycled plastic to various known plastic molding methods without limitation. As an example of the molding method, injection molding, foam injection molding, blow molding, or extrusion molding may be mentioned.

Examples of the molded article are not particularly limited, and can be applied to various molded articles using plastic without limitation. Examples of the molded article include automobiles, electrical and electronic products, communication products, daily necessities, building materials, optical components, exterior materials, and the like.

The molded article may further include one or more additives selected from the group consisting of an antioxidant, a plasticizer, an antistatic agent, a nucleating agent, a flame retardant, a lubricant, an impact enhancer, an optical brightener, an ultraviolet absorber, a pigment and a dye, if necessary, in addition to the recycled plastic according to the other embodiments.

An example of the manufacturing method of the molded article may include a step of mixing the recycled plastic according to the other embodiment and an additive well using a mixer, extrusion-molding the mixture with an extruder to produce pellets, drying the pellets, and then injecting them with an injection molding machine.

### [Advantageous Effects]

According to the present invention, there can be provided a monomer composition for synthesizing recycled plastic that can realize high purity and excellent color quality even though it is recovered through recycling by chemical decomposition of a polycarbonate-based resin, and achieve improvement in efficiency of the recovering process, a preparation method thereof, and a recycled plastic and molded product using the same.

### [DETAILED DESCRIPTION OF THE EMBODIMENTS]

Hereinafter, the present invention will be explained in detail with reference to the following examples. However, these examples are for illustrative purposes only, and the scope of the present invention is not limited thereto.

### <EXAMPLE and COMPARATIVE EXAMPLE: Preparation of recycled bisphenol A monomer composition>

### Example 1

(1. Decomposition step) 1 mol of Pretreated waste polycarbonate (PC) was dissolved in 17 mol of methylene chloride(MC), and then added together with 11 mol of ethanol (EtOH) and 0.25 mol of sodium hydroxide (NaOH) to a 3L high-pressure reactor, sodium hydrosulfite as an antioxidant was added in an amount of 0.7 wt.% relative to the total solution, the atmosphere inside the system was replaced with nitrogen, and the mixture was stirred at 60°C for 6 hours in an inactive state to proceed a PC depolymerization reaction.
(2. pH adjustment) The product of the depolymerization reaction was cooled to 30°C or less, and then, the bisphenol A was adjusted to have pH 8 by adding 10% hydrochloric acid (HCl) and water to the product.
(3. Layer separation) After that, in a state in which the water layer and the methylene chloride (MC) layer were formed, the organic layer located on the bottom side was recovered using a drain device located at the bottom end of the reactor, and the water layer located on the top side was discharged and then discarded.
(4. Distillation) After that, the recovered methylene chloride (MC) layer was subjected to a low-temperature distillation reducing pressure from 250 mbar and 20~30°C to 30 mbar and 30°C, and thus diethyl carbonate(DEC) as a by-product was separated and recovered.
(5. Purification step-Filtration) After that, the residue from which diethyl carbonate (DEC) was removed was primarily washed using methylene chloride(MC) of twice the weight of PC used at 20~30°C, and vacuum-filtered to recover bisphenol A.
(6-1. Additional purification step-Redissolving step) After that, 50g of the recovered bisphenol A and 50 g of diethyl carbonate(DEC) recovered in the 4. Distillation step were mixed at room temperature, and then the mixture was heated to 80°C with stirring, and redissolved.
(6-2. Additional purification step - Recrystallization step) The solution was cooled to 20°C to recrystallize bisphenol A, and then the resulting slurry was vacuum-filtered at 20°C to recover bisphenol A (BPA) crystals.
(7. Drying step) After that, the result was vacuum-dried in a convection oven at 100°C to prepare a recycled bisphenol A monomer composition in which recycled bisphenol A (BPA) has been recovered.

### Example 2

(1. Decomposition step) 1 mol of Pretreated waste polycarbonate (PC) was dissolved in 17 mol of methylene chloride(MC), and then added together with 11 mol of ethanol(EtOH) and 0.25 mol of sodium hydroxide(NaOH) to a 3L high-pressure reactor, sodium hydrosulfite as an antioxidant was added in an amount of 0.7 wt.% relative to the total solution, the atmosphere inside the system was replaced with nitrogen, and the mixture was stirred at 60°C for 6 hours in an inactive state to proceed a PC depolymerization reaction.
(2. pH adjustment) The product of the depolymerization reaction was cooled to 30°C or less, and then, the bisphenol A was adjusted to have pH 8 by adding 10% hydrochloric acid (HCl) and water to the product.
(3. Layer separation) After that, in a state in which the water layer and the methylene chloride (MC) layer were formed, the organic layer located on the bottom side was recovered using a drain device located at the bottom end of the reactor, and the water layer located on the top side was discharged and then discarded.
(4. Distillation) After that, the recovered methylene chloride (MC) layer was subjected to a low-temperature distillation reducing pressure from 250 mbar and 20~30°C to 30 mbar and 30°C, and thus diethyl carbonate(DEC) as a by-product was separated and recovered.
(5. Purification step-Filtration) After that, the residue from which diethyl carbonate (DEC) was removed was primarily washed using methylene chloride(MC) of twice the weight of PC used at 20~30°C, and vacuum-filtered to prepare bisphenol A.
(6-1. Additional purification step-Redissolving step) After that, 50g of the recovered bisphenol A and 50 g of diethyl carbonate(DEC) recovered in the 4. Distillation step were mixed at room temperature, and then the mixture was heated to 80°C with stirring, and redissolved.
(6-2. Additional purification step - Recrystallization step) The solution was cooled to 20°C to recrystallize bisphenol A, and then the resulting slurry was vacuum-filtered at 20°C to recover bisphenol A (BPA) crystals.
(6-3. Additional purification step-Redissolving step) After that, 50g of the recovered bisphenol A and 50 g of diethyl carbonate(DEC) recovered in the 4. Distillation step were mixed at room temperature, and then the mixture was heated to 80°C with stirring, and redissolved.
(6-4. Additional purification step - Recrystallization step) The solution was cooled to 20°C to recrystallize bisphenol A, and then the resulting slurry was vacuum-filtered at 20°C to recover bisphenol A (BPA) crystals.
(7. Drying step) After that, the result was vacuum dried in a convection oven at 100°C to prepare a recycled bisphenol A monomer composition in which recycled bisphenol A (BPA) was recovered.

### Example 3

(1. Decomposition step) 1 mol of Pretreated waste polycarbonate (PC) was dissolved in 17 mol of methylene chloride(MC), and then added together with 11 mol of ethanol (EtOH) and 0.25 mol of sodium hydroxide (NaOH) to a 3L high-pressure reactor, sodium hydrosulfite as an antioxidant was added in an amount of 0.7 wt.% relative to the total solution, the atmosphere inside the system was replaced with nitrogen, and the mixture was stirred at 60°C for 6 hours in an inactive state to proceed a PC depolymerization reaction.
(2. pH adjustment) The product of the depolymerization reaction was cooled to 30°C or less, and then, the bisphenol A was adjusted to have pH 8 by adding 10% hydrochloric acid (HCl) and water to the product.
(3. Layer separation) After that, in a state in which the water layer and the methylene chloride (MC) layer were formed, the organic layer located on the bottom side was recovered using a drain device located at the bottom end of the reactor, and the water layer located on the top side was discharged, and then discarded.
(4. Distillation) After that, the recovered methylene chloride (MC) layer was subjected to a low-temperature distillation reducing pressure from 250 mbar and 20~30°C to 30 mbar and 30°C, and thus diethyl carbonate (DEC) as a by-product was separated and recovered.
(5. Purification step-Filtration) After that, the residue from which diethyl carbonate (DEC) was removed was primarily washed using methylene chloride (MC) of twice the weight of PC used at 20~30°C, and vacuum-filtered. The filtrate was secondarily washed using water of twice the mass of PC used at a temperature of 50°C.
(6-1. Additional purification step-Redissolving step) After that, bisphenol A was added to 16.6 mol of ethanol and redissolved.
(6-2. Additional purification step-Adsorption step) After that, lignite activated carbon was added as an adsorbent in a ratio of 50 wt.% relative to waste polycarbonate, and then purified through adsorption for 3 hours, and then the lignite activated carbon was removed through filtration.
(6-3. Additional purification step - Recrystallization step) After that, 300 mol of water was added to recrystallize bisphenol A, and the resulting slurry was vacuum-filtered at 20~30°C to recover bisphenol A (BPA) crystals.
(7-1. Additional purification step-Redissolving step) After that, 50g of the recovered bisphenol A and 50 g of diethyl carbonate(DEC) recovered in the 4. Distillation step were mixed at room temperature, and then the mixture was heated to 80°C with stirring, and redissolved.
(7-2. Additional purification step - Recrystallization step) The solution was cooled to 20°C to recrystallize bisphenol A, and then the resulting slurry was vacuum-filtered at 20°C to recover bisphenol A (BPA) crystals.
(8. Drying step) After that, the result was vacuum dried in a convection oven at 100°C to prepare a recycled bisphenol A monomer composition in which recycled bisphenol A (BPA) was recovered.

### Comparative Example 1

A recycled bisphenol A monomer composition was prepared in the same manner as in Example 1, except that (6-1. Additional purification step-Redissolving step) and (6-2. Additional purification step - Recrystallization step) of Example 1 were not carried out.

### Comparative Example 2

A recycled bisphenol A monomer composition was prepared in the same manner as in Example 3, except that (7-1. Additional purification step-Redissolving step) and (7-2. Additional purification step-Recrystallization step) of Example 3 were not carried out.

### <Experimental Example>

The physical properties of the recycled bisphenol A monomer compositions obtained in the Examples and Comparative Examples were measured by the following methods, and the results are shown in Table 1 below.

### 1. BPA Purity

After the recycled bisphenol A monomer composition was dissolved in a methanol solvent at a concentration of 0.5 mg/mL under the conditions of normal pressure and 20~30°C, solid impurities in the solution were removed using a syringe filter (0.45µm), and then the purity of the bisphenol A (BPA) was analyzed by UPLC (ultra performance liquid chromatography) via Waters HPLC system using Capcell Pak C18 (Inner diameter 4.6mm, Length 50mm, Particle size 5µm). Specifically, the peak area ratio (unit: %) of bisphenol A (BPA) was measured with respect to 100% of the total UPLC peak area.

The specific UPLC measurement conditions are as follows.
① Column: Capcell Pak C18(4.6 mm ID x 50 mm L, 5µm), ② Column Temp: 40°C, ③ Injection volume: 10µℓ, ④ Flow: Mobile phase A-Acetonitrile/TFA=100/0.1%, B-Water/TFA=100/0.1%, Gradient elution - 0min A:B=2:8, 10min A:B=100:0, volume=1ml/min (total=10min), ⑤ Detector: 275nm

### 2. Ratio of impurities

UPLC analysis was carried out in the same manner as in the method for measuring the 1. BPA purity, and with respect to 100% of the total UPLC peak area, the peak area ratio (unit: %) of the BPA derivative [mono-ethylcarbonate of bisphenol-A (MEBPA; ethyl (4-(2-(4-hydroxyphenyl)propan-2-yl)phenyl)carbonate)], bis-ethylcarbonate of bisphenol-A (BEBPA; diethyl(propane-2,2-diylbis(4,1-phenylene)) bis(carbonate)), bisphenol A dimer (BPA dimer) impurities was measured according to the following Equation A. Ratio of BPA derivative impurities (%) = (Peak area of bisphenol A derivative measured by a liquid chromatography / total peak area measured by a liquid chromatography) X 100

In Equation A, the peak area of the bisphenol A derivative measured by a liquid chromatography is the sum of the peak areas of MEBPA, BEBPA, and BPA dimer.

### 3. APHA Color

For the recycled bisphenol A monomer composition, solid impurities in the solution were removed using a syringe filter (0.45 µm), and then measurement was carried out in the same manner as in the ASTM D1209 using a Hunterlab UltraScan PRO model device.

### 4. Melting point

The melting point (Tₘ) onset value of the recycled bisphenol A monomer composition was measured from the temperature vs heat flow graph obtained using a differential scanning calorimeter (Device name: DSC2920, Manufacturer: TA Instruments) device at a heating rate of 10°C/min in a temperature range of 0°C to 180°C. The melting point (Tₘ) was obtained through peak analysis of the heat curve of heat flow during the first temperature increase, and measurements were performed three times for each sample, and the average value was taken.

**[Table 1]**

| Measurement result of Experimental Example | | | | | | |
|---|---|---|---|---|---|---|
| Category | BPA purity (%) | BPA derivative impurities (%) | MEBPA and BEBPA (%) | BPA dimer (%) | APHA Color | Melting point (°C) |
| Example 1 | 99.75 | 0.25 | 0.05 | 0.2 | 36 | 156.5 |
| Example 2 | 99.88 | 0.12 | 0 | 0.12 | 27 | 156.8 |
| Example 3 | 99.91 | 0.09 | 0 | 0.09 | 6 | 156.9 |
| Comparative Example 1 | 98.82 | 0.95 | 0.48 | 0.47 | 133 | 154.8 |
| Comparative Example 2 | 99.57 | 0.35 | 0.21 | 0.14 | 17 | 156.3 |

As shown in Table 1, the recycled bisphenol A monomer compositions obtained in Examples 1 to 3 exhibited high purity of 99.75% to 99.91% and had a high melting point of 156.5°C to 156.9°C. Also, the recycled bisphenol A monomer compositions obtained in Examples 1 to 3 were measured to have a low BPA derivative impurity ratio of 0.09% to 0.25%.

On the other hand, the recycled bisphenol A monomer composition obtained in Comparative Examples 1 to 2 had a purity of 98.82% to 99.57% which was reduced as compared to that of Examples, and had a melting point of 154.8°C to 156.3°C which was lower than that of Examples. In addition, the BPA derivative impurity ratio was measured to be 0.35% to 0.95%, which was higher than that of Examples.

## Claims

1. A monomer composition for synthesizing recycled plastic, comprising an aromatic diol compound,
wherein a ratio of aromatic diol compound derivative impurities according to the following Equation 1 is 0.3% or less,
wherein the melting point of the monomer composition is 156.4°C or more, and
wherein the monomer composition is recovered from a polycarbonate-based resin: Ratio of aromatic diol compound derivative impurities (%) = (Peak area of aromatic diol compound derivative measured by a liquid chromatography / Total peak area measured by the liquid chromatography) X 100.

2. The monomer composition for synthesizing recycled plastic according to claim 1, wherein the aromatic diol compound derivative comprises one or more compounds selected from the group consisting of monoalkyl carbonate of bisphenol A, bisalkyl carbonate of bisphenol A, and bisphenol A dimer.

3. The monomer composition for synthesizing recycled plastic according to claim 2, wherein a ratio of the monoalkyl carbonate of bisphenol A and the bisalkyl carbonate of bisphenol A according to the following Equation 2 is 0.1% or less: Ratio of the monoalkyl carbonate of bisphenol A and the bisalkyl carbonate of bisphenol A (%) = (Peak area of monoalkyl carbonate of bisphenol A and bisalkyl carbonate of bisphenol A measured by a liquid chromatography / Total peak area measured by the liquid chromatography) X 100.

4. The monomer composition for synthesizing recycled plastic according to claim 2, wherein a ratio of the bisphenol A dimer according to the following Equation 3 is 0.3% or less: Ratio of the bisphenol A dimer (%) = (Peak area of the bisphenol A dimer measured by a liquid chromatography / Total peak area measured by the liquid chromatography) X 100.

5. The monomer composition for synthesizing recycled plastic according to claim 1, wherein the monomer composition for synthesizing recycled plastic has an APHA color value of 60 or less, which is measured according to ASTM D 1209.

6. The monomer composition for synthesizing recycled plastic according to claim 1, wherein the monomer composition for synthesizing recycled plastic has an aromatic diol compound purity of 99.6% or more.

7. A method for preparing a monomer composition for synthesizing recycled plastic, the method comprising the steps of:
subjecting a polycarbonate-based resin to a depolymerization reaction;
separating a carbonate-based compound from depolymerization reaction product;
dissolving the depolymerization reaction product from which the carbonate-based compound has been separated in a solvent containing the separated carbonate-based compound; and
recrystallizing aromatic diol compound from the dissolved solution.

8. The method for preparing a monomer composition for synthesizing recycled plastic according to claim 7, wherein:
the dissolving the depolymerization reaction product from which the carbonate-based compound has been separated in the solvent containing the separated carbonate-based compound is carried out at a temperature of 0°C to 200°C.

9. The method for preparing a monomer composition for synthesizing recycled plastic according to claim 7, wherein:
in the dissolving the depolymerization reaction product from which the carbonate-based compound has been separated in the solvent containing the separated carbonate-based compound,
the addition amount of the solvent containing the carbonate-based compound is 10 part by weight or more, based on 100 parts by weight of the aromatic diol compound contained in the depolymerization reaction product from which the carbonate-based compound has been separated.

10. The method for preparing a monomer composition for synthesizing recycled plastic according to claim 7, wherein:
in the recrystallizing the aromatic diol compound from the dissolved solution,
the concentration of the aromatic diol compound contained in the solution is 90% by weight or less.

11. The method for preparing a monomer composition for synthesizing recycled plastic according to claim 7, wherein:
the recrystallizing the aromatic diol compound from the dissolved solution comprises cooling the dissolved solution.

12. The method for preparing a monomer composition for synthesizing recycled plastic according to claim 7, wherein:
the dissolving the depolymerization reaction product from which the carbonate-based compound has been separated in the solvent containing the separated carbonate-based compound, and
the recrystallizing the aromatic diol compound from the dissolved solution,
are repeated two or more times.

13. The method for preparing a monomer composition for synthesizing recycled plastic according to claim 7, further comprising:
adding an adsorbent to the depolymerization reaction product from which the carbonate-based compound has been separated, to perform an adsorption purification and then removing the adsorbent,
between the separating the carbonate-based compound from the depolymerization reaction product; and the dissolving the depolymerization reaction product from which the carbonate-based compound has been separated in the solvent containing the separated carbonate-based compound.

14. The method for preparing a monomer composition for synthesizing recycled plastic according to claim 7, wherein:
the depolymerization reaction of the polycarbonate-based resin is carried out in the presence of an alcohol.

15. The method for preparing a monomer composition for synthesizing recycled plastic according to claim 14, wherein:
the amount of the alcohol is 5 to 15 moles relative to 1 mole of the polycarbonate-based resin.

16. The method for preparing a monomer composition for synthesizing recycled plastic according to claim 7, wherein the carbonate-based compound includes dialkyl carbonate or diaryl carbonate.

17. The method for preparing a monomer composition for synthesizing recycled plastic according to claim 7, further comprising:
washing the depolymerization reaction product from which the carbonate-based compound has been separated,
between the separating the carbonate-based compound from the depolymerization reaction product, and the dissolving the depolymerization reaction product from which the carbonate-based compound has been separated in the solvent containing the separated carbonate-based compound.

18. A method for preparing a monomer composition for synthesizing recycled plastic, the method comprising the steps of:
subjecting a polycarbonate-based resin to a depolymerization reaction;
dissolving depolymerization reaction product in a solvent containing a carbonate-based compound; and
recrystallizing aromatic diol compound from the dissolved solution.

19. A recycled plastic comprising a reaction product of the monomer composition for synthesizing recycled plastic according to claim 1 and a comonomer.

20. A molded product comprising the recycled plastic according to claim 19.
